# FASCICULE DE BREVET EUROPEEN

(11) **EP 0 322 323 B1**
(45) Date de publication et mention de la délivrance du brevet: **10.03.1993**
(21) Numéro de dépôt: 88420424.9
(22) Date de dépôt: 16.12.1988
(51) Int. Cl.: C07D 301/32, C07D 303/04

(54) **Procédé pour séparer l'oxyde d'éthylène d'impuretés aldéhydiques**
Verfahren zur Abtrennung von aldehydischen Verunreinigungen aus Ethylenoxid
Process for separating aldehydic impurities from ethylene oxide

(30) Priorité: 22.12.1987 FR 8718246
(43) Date de publication de la demande: 28.06.1989
(73) Titulaire: ELF ATOCHEM S.A., 92800 Puteaux (FR)
(72) Inventeur: Delannoy, Francis, F-69310 Pierre-Benite (FR); Letray, Gérard, F-76620 Le Havre (FR)

(56) Documents cités:
- US-A- 2 615 901
- US-A- 3 418 338
- US-A- 4 134 797

## Description

La présente invention concerne un procédé pour séparer l'oxyde d'éthylène d'impuretés aldéhydiques : formaldéhyde et aussi acétaldéhyde.

L'oxyde d'éthylène accompagné desdites impuretés résulte ou dérive essentiellement de la fabrication de l'oxyde d'éthylène par oxydation catalytique en phase gazeuse de l'éthylène par l'oxygène moléculaire.

De façon connue, l'isolement de l'oxyde d'éthylène, à partir du mélange gazeux tel qu'issu de la zone d'oxydation catalytique de l'éthylène, se fait habituellement en plusieurs stades comprenant :
(a) : une absorption à l'eau consistant à mettre en contact ce mélange gazeux avec de l'eau pour obtenir une solution aqueuse d'oxyde d'éthylène contenant le plus couramment environ 2 à 3 % d'oxyde d'éthylène en poids, les impuretés aldéhydiques et, à l'état dissous, des composés normalement gazeux dont notamment le CO₂,
(b) : une désorption consistant à soumettre la solution diluée d'oxyde d'éthylène obtenue en (a) à un entrainement à l'aide de la vapeur d'eau pour obtenir un mélange gazeux comprenant à côté le plus souvent de 30 % à 60 % en poids d'oxyde d'éthylène, du CO₂ et des impuretés aldéhydiques, formaldéhyde et acétaldéhyde.
(c) : une réabsorption par l'eau de l'oxyde d'éthylène contenu dans le mélange gazeux obtenu en (b) pour former une solution aqueuse d'oxyde d'éthylène comprenant le plus fréquemment environ 5 % à 25 % d'oxyde d'éthylène en poids et encore du CO₂ et des impuretés aldéhydiques.
(d) : une distillation de la solution aqueuse d'oxyde d'éthylène obtenue en (c), pour obtenir d'une part l'oxyde d'éthylène avec peu ou pratiquement pas d'eau, et d'autre part un courant aqueux pratiquement exempt d'oxyde d'éthylène.

L'oxyde d'éthylène obtenu ainsi en (d) contient une quantité d'impuretés aldéhydiques, formaldéhyde et acétaldéhyde, nettement trop importante pour que soit atteinte la qualité que peut requérir l'industrie.

Le brevet des Etats Unis d'Amérique US-4134797 propose un procédé pour diminuer la teneur en impuretés aldéhydiques dans l'oxyde d'éthylène, dans lequel un courant aqueux essentiellement exempt d'oxyde d'éthylène est évacué au bas de la colonne employée. Cet épuisement dudit courant aqueux en oxyde d'éthylène est garanti par l'introduction dans la colonne de vapeur d'eau comme fluide d'entraînement. L'oxyde d'éthylène sort de la colonne simultanément à trois niveaux différents, sous forme respectivement d'un courant d'oxyde d'éthylène riche en formaldéhyde, d'un courant riche en acétaldéhyde, et enfin d'un courant d'oxyde d'éthylène qui peut contenir d'environ 0,001 % à 0,035 % d'impuretés aldéhydiques. Ce dernier courant, n'est obtenu qu'au prix d'un processus distillatoire très complexe qui, selon les exemples fournis, n'en laisse pas moins 25 % à 40 % environ de l'oxyde d'éthylène sous forme non purifiée.

Le brevet US 2615901 concerne un procédé dans lequel on sépare l'eau de l'oxyde d'éthylène et de l'acétaldéhyde puis le mélange d'oxyde d'éthylène et d'acétaldéhyde est flashé, le restant (oxyde plus acétaldéhyde) est hydraté pour extraire sélectivement l'oxyde d'éthylène sous forme de glycol.

Le brevet US 3418338 concerne une distillation extractive d'une solution à 0,5 % d'oxyde d'éthylène contenant seulement du formaldéhyde.

Le procédé de l'invention convient à la séparation de l'oxyde d'éthylène du formaldéhyde et de l'acétaldéhyde à partir d'oxyde d'éthylène comprenant, à côté de ces impuretés, peu ou beaucoup d'eau indifféremment. Il peut être exécuté de façon à assurer une récupération sous forme purifiée de la presque totalité de l'oxyde d'éthylène. Il n'implique qu'un simple et unique stade distillatoire. Il convient à la séparation du formaldéhyde aussi bien qu'à celle de l'acétaldéhyde. Il trouve son principal intérêt à la séparation à la fois du formaldéhyde et de l'acétaldéhyde.

Il conduit à l'oxyde d'éthylène séparé du formaldéhyde et, simultanément, de l'acétaldéhyde, par exemple à partir :
- du courant d'oxyde d'éthylène impur résultant d'un stade tel que (d) de la technique connue ou bien des courants d'oxyde d'éthylène impur mentionnés dans le brevet US-4134797 déjà cité, courants auxquels est ajoutée de l'eau si nécessaire.
- du courant d'oxyde d'éthylène résultant d'un stade tel que (b), en particulier du courant liquide sortant du dernier échangeur lors de la condensation progressive du courant gazeux issu en tête de la colonne de désorption dans un stade (b) quand ladite condensation est réalisée dans deux ou trois échangeurs de chaleur disposés en série de façon analogue à celle décrite par exemple dans la demande de brevet européen EP-0139601. Un procédé de fabrication d'oxyde d'éthylène dans lequel le processus d'isolement de l'oxyde d'éthylène à partir du courant gazeux issu de la zone d'oxydation catalytique comprend une absorption selon (a), une désorption selon (b) avec condensation progressive du courant gazeux de tête définie ci-dessus et une séparation de l'oxyde d'éthylène du formaldéhyde et de l'acétaldéhyde selon l'invention, est ainsi rendu particulièrement attractif pour sa simplicité et son économie. Il constitue un second objet de l'invention dont le premier est :
   un procédé pour séparer l'oxyde d'éthylène du formaldéhyde et de l'acétaldéhyde à partir d'oxyde d'éthylène impur contenant lesdits aldéhydes à titre d'impuretés et de l'eau, par distillation dudit oxyde d'éthylène impur dans une colonne avec reflux, caractérisé en ce que ladite distillation est réalisée dans des conditions telles que le courant liquide qui sort en bas de la colonne contient l'eau initialement présente dans l'oxyde d'éthylène impur et de l'oxyde d'éthylène en quantité pondérale comprise entre 0,15 fois et 3 fois celle de l'eau, tandis que l'oxyde d'éthylène résultant de la séparation du formaldéhyde et de l'acétaldéhyde sort en haut de la colonne. La présente invention concerne aussi un procédé selon la revendication 12.

Bien que le procédé de l'invention ainsi défini soit applicable à la séparation visée à partir d'oxyde d'éthylène contenant jusqu'à environ 75 % d'eau en poids, il est le plus intéressant en pratique lorsque cette proportion d'eau est égale ou inférieure à environ 20 %, voire 10 %, compte tenu de la facilité avec laquelle il est possible d'amener la teneur en eau au niveau de ces dernières valeurs. La quantité d'eau dans l'oxyde d'éthylène impur n'est de préférence pas inférieure à environ 0,5 % en poids pour que le fonctionnement de la colonne reste aisé.

Il est aussi normalement avantageux que corresponde à la séparation visée un courant liquide sortant au bas de la colonne ne contenant qu'une proportion limitée de l'oxyde d'éthylène présent dans le produit impur comme le permet aisément l'invention. De préférence la quantité pondérale d'oxyde d'éthylène dans ledit courant est le plus souvent comprise entre 0,3 fois et 1,5 fois celle de l'eau.

La quantité pondérale d'impuretés aldéhydiques, formal - déhyde et acétaldéhyde, dans l'oxyde d'éthylène impur, rapportée à celle de l'oxyde d'éthylène, peut varier dans d'assez grandes limites, par exemple entre celles énoncées dans le brevet des Etats Unis d'Amérique US-4134797. Le plus fréquemment en pratique l'oxyde d'éthylène impur contient en poids, par rapport à l'oxyde d'éthylène contenu, environ 0,005 % à 0,2 % desdits aldéhydes, le rapport entre le formaldéhyde et l'acétaldéhyde n'étant pas critique pour l'invention.

Dans le cas particulier où la méthode qui vient d'être décrite est appliquée à la séparation de l'oxyde d'éthylène d'impuretés aldéhydiques à partir d'oxyde d'éthylène du type de celui qui résulte de la condensation progressive évoquée plus haut lors de la citation de la demande de brevet européen EP-0139601. Ledit oxyde d'éthylène impur peut contenir, en poids, au moins environ 95 % et souvent plus de 97 % d'oxyde d'éthylène et généralement entre environ 0,005 % à 0,05 % d'impuretés aldéhydiques.

Lorsque des composés normalement à l'état gazeux, tel qu'essentiellement le CO₂, se trouvent à l'état dissous dans l'oxyde d'éthylène impur, ils en sont de préférence pratiquement chassés de façon connue préalablement à la distillation, par exemple par dégazage ou strippage par simple chauffage sous pression de l'oxyde d'éthylène impur.

La séparation de l'oxyde d'éthylène des impuretés aldéhydiques dans le procédé de l'invention est réalisée dans une colonne classique de distillation, par exemple dans une colonne à plateaux comportant un nombre de plateaux théoriques égale à titre indicatif à environ 50 mais pouvant être compris selon les cas par exemple entre environ 30 et 60, qui fonctionne à une pression absolue moyenne comprise le plus souvent entre environ 2,5 bars et 5 bars de sorte que dans les conditions préférées d'éxécution la proportion pondérale oxyde d'éthylène/eau recherchée au bas de la colonne puisse être respectée à une température comprise le plus souvent entre environ 40°C et 60°C dans cette zone. L'oxyde d'éthylène impur est introduit dans la colonne à un niveau situé de préférence entre environ la moitié et les trois-quarts du nombre de plateaux théoriques comptés à partir du haut. Les calories nécessaires sont fournies à la colonne par exemple en assurant le rebouillage en pied à l'aide de rebouilleurs alimentés par de la vapeur d'eau ou par un courant adéquat dans une unité de production d'oxyde d'éthylène comme le courant gazeux issu de la tête de la colonne de désorption mentionnée plus haut sous (b). L'oxyde d'éthylène résultant de la séparation des impuretés aldéhydiques sort en haut de la colonne sous forme de courant gazeux, qui est condensé. Une partie du condensat retourne dans la colonne à titre de reflux en tête de colonne tandis que la partie complémentaire non refluée constitue le courant d'oxyde d'éthylène produit.

Le rapport oxyde d'éthylène reflué/oxyde d'éthylène produit peut varier entre des limites relativement éloignées. Il est en général compris entre 1/1 et 9/1 et le plus fréquemment entre 1,5/1 et 6/1.

Ledit oxyde d'éthylène produit ne renferme plus, à côté d'une quantité d'eau ne dépassant pas le plus souvent 0,002 % en poids, qu'une quantité d'impuretés aldéhydiques : formaldéhyde et acétaldéhyde, habituellement inférieure à environ 0,0025 %, souvent à environ 0,0015 %.

La formation de produits de transformation de l'oxyde d'éthylène, comme les glycols, est pratiquement négligeable dans le procédé de l'invention. Le courant liquide sortant au bas de la colonne de distillation dans le procédé de l'invention peut être traité de sorte par exemple à transformer de façon connue l'oxyde d'éthylène en glycol puis à séparer les impuretés aldéhydiques du glycol pour les évacuer hors du circuit de production.

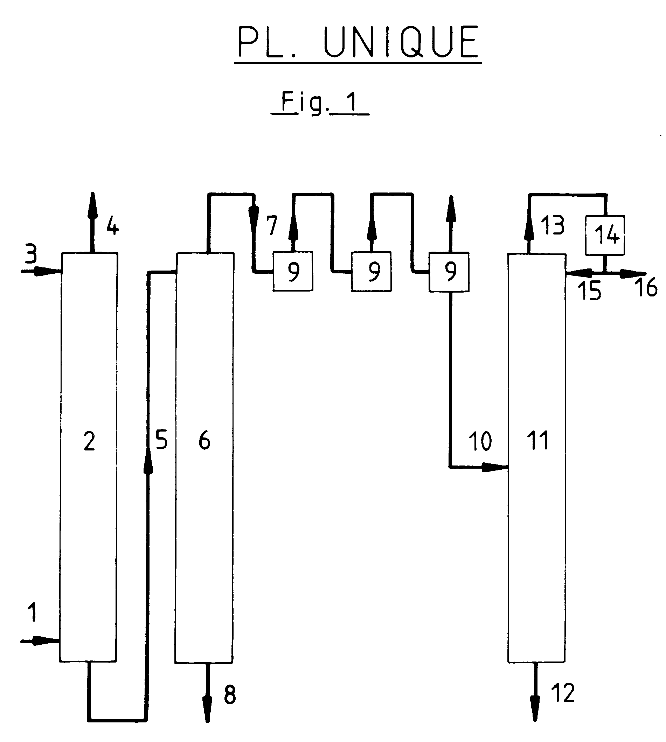

La figure 1 de la planche unique illustre schématiquement le processus d'isolement de l'oxyde d'éthylène extrêmement simple que permet le procédé de l'invention dans un procédé de fabrication d'oxyde d'éthylène par oxydation catalytique de l'éthylène en phase gazeuse par l'oxygène.

Sur le schéma, 2 et 6 désignent respectivement une colonne d'absorption du stade (a) et une colonne de désorption du stade (b) fonctionnant dans des conditions connues tandis que 11 désigne la colonne de séparation de l'oxyde d'éthylène des impuretés aldéhydiques adaptée à la réalisation de l'invention.

Le courant gazeux 1 en provenance de la zone d'oxydation catalytique de l'éthylène pénètre à un niveau inférieur dans la colonne d'absorption 2 fonctionnant sous pression et comportant plusieurs étages théoriques ou dispositifs de contact gaz-liquide, pour être mis en contact à contre-courant avec le courant d'absorption 3 constitué en majeure partie d'eau et entrant dans la colonne 2, à un niveau supérieur. Le courant gazeux 4 qui sort au sommet de 2 contient des traces d'oxyde d'éthylène, de l'éthylène, de l'oxygène, du CO₂ et des gaz inertes. De façon connue ce courant gazeux, après diminution de sa teneur en CO₂, est recyclé à l'étape d'oxydation de l'éthylène. Le courant aqueux liquide 5 sortant au bas de la colonne 2 renferme l'oxyde d'éthylène à une concentration souvent peu éloignée d'environ 2 % à 3 % en poids, des gaz dissous, dont surtout le CO₂, des impuretés aldéhydiques.

Le courant 5 pénètre à un niveau supérieur dans la colonne 6 pour que l'oxyde d'éthylène qu'il renferme soit extrait, à l'aide de la vapeur d'eau, sous la forme du courant gazeux 7 renfermant le plus souvent environ 30 % à 60 % en poids d'oxyde d'éthylène, de l'eau et des impuretés aldéhydiques. Ce courant peut pratiquement ne pas contenir de gaz dissous si le courant 5 est traité par exemple selon le procédé décrit dans la demande de brevet européen EP-0149585. Il contient par contre les impuretés aldéhydiques. Le courant aqueux 8 sortant au bas de la colonne 6 est refroidi et sert pour l'essentiel de courant d'absorption 3.

Le courant 7 est progressivement condensé dans deux ou trois échangeurs de chaleur 9.

La colonne 6 et les échangeurs 9, sur le plan de la structure comme sur celui du fonctionnement, sont tels que décrits dans la demande de brevet européen EP-0139601.

Le courant liquide 10 sortant du dernier échangeur et duquel, si nécessaire, les gaz encore dissous peuvent être éliminés de façon connue par exemple par dégazage comme évoqué plus haut, contient en poids, comme déjà indiqué, généralement plus d'environ 95 %, souvent au moins 97 %, d'oxyde d'éthylène, jusqu'à 0,2 % le plus souvent entre 0,005 % et 0,05 % d'impuretés aldéhydiques à côté de l'eau.

Il entre dans la colonne de distillation 11 qui assure la séparation de l'oxyde d'éthylène des impuretés aldéhydiques dans la structure et selon le fonctionnement déjà décrits.

12 désigne le courant liquide sortant au bas de la colonne 11. Il renferme l'oxyde d'éthylène et l'eau dans un rapport pondéral correspondant à la définition de l'invention.

13 désigne le courant gazeux sortant en haut de la colonne 11 qui est constitué de l'oxyde d'éthylène tel qu'il résulte de la séparation selon l'invention. Il est condensé en 14 de sorte à former d'une part le reflux liquide 15 introduit dans la colonne 11 à son niveau supérieur et d'autre part le courant 16 d'oxyde d'éthylène produit.

Les exemples ci-après donnés à titre indicatif mais non limitatif illustrent l'invention en comparaison avec l'art de la technique.

Dans les exemples, les données pondérales supérieures à 1000 kg sont arrondies au kilogramme et référence est faite au schéma de la figure 1 de la planche unique.

### Exemple 1 :

Dans un procédé de fabrication d'oxyde d'éthylène par oxydation catalytique en phase gazeuse de l'éthylène par l'oxygène, qui comporte un processus d'isolement de l'oxyde d'éthylène selon le schéma simplifié de la figure 1, la solution aqueuse diluée d'oxyde d'éthylène obtenue au bas de la colonne d'absorption 2 est débarassée de la plus grande partie des gaz dissous en elle par application de la technique décrite dans la demande de brevet européen EP-0149585 et non figurée.

Elle est ensuite soumise dans colonne 6 à une désorption de l'oxyde d'éthylène avec condensation progressive du courant gazeux qui en résulte, dans trois échangeurs de chaleur 9 selon la technique décrite dans la demande de brevet européen EP-0139601.

Le courant gazeux qui sort en haut de la colonne 6 contient des impuretés aldéhydiques et est essentiellement constitué d'oxyde d'éthylène et d'eau.

Bien que cela puisse ne pas constituer une nécessité, le courant liquide qui sort du dernier des trois échangeurs, est débarrassé des gaz qui pourraient encore être dissous en lui, par évacuation de ceux-ci par chauffage dans une colonne, non figurée, de technologie bien connue. Le courant liquide qui entre au niveau supérieur de cette colonne de dégazage en sort par le bas, à une température et à une pression absolue égales dans le cas présent à 63°C et 5,6 bars respectivement. Il constitue l'oxyde d'éthylène impur qui a un débit de 5755 kg/h et contient, en poids, 97,4 % d'oxyde d'éthylène et 2,6 % d'eau, valeurs non corrigées des 0,011 % d'impuretés aldéhydiques se répartissant en 0,0085 % d'acétaldéhyde et 0,0025 % de formaldéhyde, ni des autres produits présents à l'état de traces.

Il entre dans la colonne 11, qui comprend 50 plateaux théoriques, au niveau du 30ème plateau compté à partir du sommet.

La colonne 11 qui fonctionne à une pression absolue moyenne de 2,7 bars est pourvue en calories de manière classique par échange de chaleur indirect de la vapeur d'eau avec le liquide en pied dont le rebouillage est ainsi assuré. La température du liquide au bas de la colonne est égale à 42°C. Le débit du courant liquide sortant au bas de la colonne est de 290 kg/h. Ledit courant contient, en poids, 51,5 % d'eau, 48,3 % d'oxyde d'éthylène et est dirigé vers une fabrication de glycol.

Au sommet de la colonne 11, sort, à une température égale à 35,4°C, un courant gazeux constitué d'oxyde d'éthylène ne renfermant qu'une quantité d'impuretés aldéhydiques variant entre environ 0,0015 et 0,0020 % en poids dont pas plus d'environ 0,0005 % de formaldéhyde. Les traces d'eau que contient ce courant ne sont pas prises en compte ici.

Une fraction du liquide résultant de la condensation du courant gazeux ci-dessus est retournée dans la colonne à titre de reflux au niveau supérieur de la colonne 11 tandis que la fraction complémentaire, de débit égal à 5465 kg/h constitue l'oxyde d'éthylène produit, le rapport pondéral oxyde d'éthylène reflué/oxyde d'éthylène produit étant ici égal à 1,5/1.

### Exemple 2 (comparatif)

Dans un processus d'isolement de l'oxyde d'éthylène à partir du courant gazeux issu de la zone d'oxydation catalytique en phase vapeur par l'oxygène, qui comprend les stades (a), (b), (c) et (d) de la technique connue telles que définies en début de texte, le courant d'oxyde d'éthylène résultant de (d) contient outre des traces d'eau, 0,01 % d'impuretés aldéhydiques.

Ce courant, dont le débit est de 5700 kg/h, est distillé dans la colonne 11 de l'exemple 1 dont les conditions de pression absolue moyenne et de reflux sont conservées. Du pied de cette colonne sort, à une température égale à 40°C et à un débit horaire de 143 kg/h, un courant liquide essentiellement constitué d'oxyde d'éthylène et qui est tel que la quantité horaire d'oxyde d'éthylène ainsi évacuée en pied de colonne est quasiment la même que dans l'exemple 1.

Le courant d'oxyde d'éthylène produit a un débit égal à 5557 kg/h. Il est constitué d'oxyde d'éthylène bien entendu pratiquement anhydre mais qui contient encore une quantité d'impuretés aldéhydiques égale à 0,0045 % en poids et donc environ 2,5 à 3 fois supérieure à celle de l'oxyde d'éthylène produit dans l'exemple 1. Le formaldéhyde initialement présent dans l'oxyde d'éthylène impur se retrouve pratiquement en totalité dans l'oxyde d'éthylène produit.

### Exemple 3 :

L'exemple 1 est répété en évacuant en pied de la colonne 11, à une température qui est égale à 40°C, 374 kg/h d'un courant liquide comprenant, en poids 59,9 % d'oxyde d'éthylène et 39,9 % d'eau.

L'oxyde d'éthylène produit, de débit égal à 5381 kg/h, contient une quantité d'impuretés aldéhydiques qui reste inférieure à 0,0015 % dont constamment moins de 0,0005 % de formaldéhyde.

### Exemple 4 :

En opérant comme dans l'exemple 1 mais en soutirant au bas de la colonne 11, à une température égale à 44°C, 250 kg/h d'un courant liquide contenant en poids 40 % d'oxyde d'éthylène et 59,8 % d'eau, l'oxyde d'éthylène produit au débit de 5505 kg/h ne contient en moyenne que 0,0022 % d'impuretés aldéhydiques dont une fois encore 0,0005 % de formaldéhyde au plus.

### Exemple 5 :

5755 kg/h d'oxyde d'éthylène impur contenant en poids 94,8 % d'oxyde d'éthylène, 5,2 % d'eau et 0,011 % d'impuretés aldéhydiques, formaldéhyde et acétaldéhyde, sont distillés dans la colonne 11 comme dans l'exemple 1 à cela près que le courant soutiré au bas de cette colonne a cette fois un débit égal à 600 kg/h et qu'il contient en poids 50 % d'eau et 50 % d'oxyde d'éthylène.

L'oxyde d'éthylène produit contient en poids, à nouveau moins de 0,0015 % d'impuretés aldéhydiques dont le tiers est du formaldéhyde.

Dans les mêmes conditions que ci-dessus, la distillation d'un oxyde d'éthylène impur contenant aussi 0,011 % d'impuretés aldéhydiques mais seulement environ 0,001 % de formaldéhyde, conduit à un oxyde d'éthylène produit contenant moins de 0,0010 % d'impuretés aldéhydiques et pratiquement totalement débarassé de formaldéhyde.

### Exemple 6 :

De l'oxyde d'éthylène impur contient en poids, en plus de l'oxyde d'éthylène, 1,2 % d'eau et 0,028 % d'impuretés aldéhydiques se répartissant en 0,026 % d'acétaldéhyde et 0,002 % de formaldéhyde.

Il est introduit à raison de 8102 kg/h dans la colonne 11 de l'exemple 1 pour être distillé à une pression absolue moyenne de 4,25 bars. En bas de la colonne sortent à 58°C, 220 kg/h d'un courant liquide contenant 44,5 % d'eau et 54,5 % d'oxyde d'éthylène. Le courant gazeux issu à 50°C en haut de la colonne au débit de 51233 kg/h est constitué d'oxyde d'éthylène pratiquement exempt d'eau comme dans tous les exemples précédents et ne contenant que moins de 0,0015 % d'impuretés aldéhydiques.

Après condensation de ce courant gazeux, 7882 kg/h d'oxyde d'éthylène produit sont recueillis, le rapport oxyde d'éthylène reflué/oxyde d'éthylène produit étant ici égal à 5,5/1.

### Exemple 7 (comparatif)

L'exemple 6 est repris en opérant dans la colonne 11 de façon à ce que l'eau contenue dans l'oxyde d'éthylène impur soit une fois encore présente dans le liquide soutiré en son pied, mais avec ici une quantité d'oxyde d'éthylène telle que le rapport pondéral de l'oxyde d'éthylène à l'eau soit égal à 20.

L'oxyde d'éthylène produit renferme non seulement une quantité d'impuretés aldéhydiques conséquente et égale en poids à 0,005 % mais encore le formaldéhyde représente la moitié de cette teneur, donc la presque totalité du formaldéhyde présent dans l'oxyde d'éthylène impur.

## Revendications

1. Procédé pour séparer l'oxyde d'éthylène du formaldéhyde et de l'acétaldéhyde à partir d'oxyde d'éthylène impur contenant lesdits aldéhydes à titre d'impuretés et de l'eau par distillation dudit oxyde d'éthylène impur dans une colonne avec reflux, caractérisé en ce que ladite distillation est réalisée dans des conditions telles que le courant liquide qui sort en bas de la colonne contient l'eau présente dans l'oxyde d'éthylène impur et de l'oxyde d'éthylène en quantité pondérale comprise entre 0,15 fois et 3 fois celle de l'eau, tandis que l'oxyde d'éthylène résultant de la séparation du formaldéhyde et de l'acétaldéhyde sort en haut de la colonne.

2. Procédé selon la revendication 1, caractérisé en ce que le rapport pondéral de la quantité d'oxyde d'éthylène à celle de l'eau dans le courant liquide qui sort en bas de la colonne est compris entre 0,3 et 1,5.

3. Procédé selon l'une des revendications 1 ou 2, caractérisé en ce que la quantité d'impuretés aldéhydiques, formaldéhyde et acétaldéhyde dans l'éthylène impur, rapportée à celle d'oxyde d'éthylène,est comprise en poids entre 0,005 % et 0,02 %.

4. Procédé selon l'une quelconque des revendications 1 à 3, caractérisé en ce que l'oxyde d'éthylène impur contient une quantité d'oxyde d'éthylène égale ou supérieure à 80 % en poids.

5. Procédé selon l'une quelconque des revendications 1 à 4, caractérisé en ce que l'oxyde d'éthylène impur contient une quantité d'eau égale ou supérieure à 0,5 % en poids.

6. Procédé selon l'une quelconque des revendications 1 à 5, caractérisé en ce que la colonne fonctionne à une pression absolue moyenne comprise entre 2,5 bars et 5 bars.

7. Procédé selon l'une quelconque des revendications 1 à 6, caractérisé en ce que la température en bas de la colonne est comprise entre 40°C et 60°C.

8. Procédé selon l'une quelconque des revendications 1 à 7, caractérisé en ce que la colonne comporte un nombre de plateaux théoriques compris entre 30 et 60.

9. Procédé selon l'une des revendications 1 à 8, caractérisé en ce que l'oxyde d'éthylène impur entre dans le colonne à un niveau situé entre la moitié et les trois-quarts du nombre de plateaux théoriques compté à partir du haut de la colonne.

10. Procédé selon la revendications 9, caractérisé en ce que le reflux consiste en une fraction condensée de l'oxyde d'éthylène séparé des impuretés aldéhydiques telle que le rapport pondéral de l'oxyde d'éthylène reflué à celui qui ne l'est pas est compris entre 1 et 9.

11. Procédé selon la revendication 10, caractérisé en ce que le rapport pondéral de l'oxyde d'éthylène reflué à celui qui ne l'est pas est compris entre 1,5 et 6.

12. Procédé d'isolement de l'oxyde d'éthylène séparé des impuretés aldéhydiques à partir du courant gazeux issu de la zone d'oxydation catalytique en phase gazeuse de l'éthylène par l'oxygène, qui comprend
(a) - une absorption à l'eau par mise en contact dudit courant gazeux avec un courant essentiellement aqueux pour obtenir une solution diluée d'oxyde d'éthylène,
(b) - une désorption consistant à soumettre la solution aqueuse diluée d'oxyde d'éthylène obtenue en (a) à un entrainement à l'aide de vapeur d'eau dans une colonne fonctionnant à une pression absolue moyenne comprise entre 1,5 bars et 6 bars, pour obtenir en pied de la colonne un courant aqueux essentiellement débarassé d'oxyde d'éthylène et en tête de colonne un courant gazeux qui comprend l'oxyde d'éthylène, et qui est progressivement condensé dans deux ou trois échangeurs de chaleur disposés en série fonctionnant à une pression absolue moyenne comprise entre 1,5 bars et 6 bars, pour le dernier desquels la température de la source froide est comprise entre 5°C et une température maximale inférieure de 5°C à la température de condensation de l'oxyde d'éthylène pur à la pression considérée,
(c) - une distillation du courant liquide sortant du dernier des échangeurs de (b),
caractérisé en ce que (c) est réalisé comme dans l'une quelconque des revendications 1 à 11, dans lesquelles l'oxyde d'éthylène impur est le courant liquide sortant du dernier des deux ou trois échangeurs en (b).

13. Procédé selon la revendication 12, caractérisé en ce que l'oxyde d'éthylène impur contient en poids 95 % d'oxyde d'éthylène ou plus et 0,005 % à 0,05 % d'impuretés aldéhydiques, formaldéhyde et acétaldéhyde.

## Claims

1. Process for separating ethylene oxide from formaldehyde and from acetaldehyde starting from impure ethylene oxide containing the said aldehydes as impurities and water, by distillation of said impure ethylene oxide in a column with reflux, characterised in that the said distillation is carried out under conditions such that the liquid stream which leaves at the bottom of the column contains the water present in the impure ethylene oxide and ethylene oxide in a quantity by weight of between 0.15 times and 3 times that of the water, while the ethylene oxide resulting from the separation from formaldehyde and from acetaldehyde leaves at the top of the column.

2. Process according to Claim 1, characterised in that the weight ratio of the quantity of ethylene oxide to that of water in the liquid stream leaving at the bottom of the column is between 0.3 and 1.5.

3. Process according to either of Claims 1 and 2, characterised in that the quantity of aldehyde impurities, formaldehyde and acetaldehyde in the impure ethylene, is between 0.005 % and 0.02 % by weight, relative to that of ethylene oxide.

4. Process according to any one of Claims 1 to 3, characterised in that the impure ethylene oxide contains a quantity of ethylene oxide equal to or greater than 80 % by weight.

5. Process according to any one of Claims 1 to 4, characterised in that the impure ethylene oxide contains a quantity of water equal to or greater than 0.5 % by weight.

6. Process according to any one of Claims 1 to 5, characterised in that the column operates at an average absolute pressure of between 2.5 bars and 5 bars.

7. Process according to any one of Claims 1 to 6, characterised in that the temperature at the bottom of the column is between 40°C and 60°C.

8. Process according to any one of Claims 1 to 7, characterised in that the column comprises a number of theoretical plates which is between 30 and 60.

9. Process according to one of Claims 1 to 8, characterised in that the impure ethylene oxide enters the column at a level situated between one half and three quarters of the number of theoretical plates, counting from the top of the column.

10. Process according to Claim 9, characterised in that the reflux consists of a condensed fraction of the ethylene oxide separated from the aldehyde impurities such that the weight ratio of the ethylene oxide which is refluxed to that which is not is between 1 and 9.

11. Process according to Claim 10, characterised in that the weight ratio of the ethylene oxide which is refluxed to that which is not is between 1.5 and 6.

12. Process for isolating the ethylene oxide separated from the aldehyde impurities from the gas stream originating from the zone of gas-phase catalytic oxidation of ethylene oxide using oxygen, which comprises
(a) - an absorption in water by bringing the said gas stream into contact with an essentially aqueous stream to obtain a dilute solution of ethylene oxide.
(b) - a desorption consisting in subjecting the dilute aqueous solution of ethylene oxide obtained in (a) to an entrainment with steam in a column operating at an average absolute pressure of between 1.5 bars and 6 bars, to obtain, at the foot of the column, an aqueous stream essentially freed from ethylene oxide and, at the head of the column, a gas stream which comprises ethylene oxide and which is progressively condensed in two or three heat exchangers arranged in series, operating at an average absolute pressure of between 1.5 bars and 6 bars, in the last of which the temperature of the cold source is between 5°C and a maximum temperature which is 5°C lower than the condensation temperature of pure ethylene oxide at the pressure in question,
(c) - a distillation of the liquid stream leaving the last of the exchangers in (b),
characterised in that (c) is carried out as in any one of Claims 1 to 11, in which the impure ethylene oxide is the liquid stream leaving the last of the two or three exchangers in (b).

13. Process according to Claim 12, characterised in that the impure ethylene oxide contains by weight 95 % of ethylene oxide or more and 0.005 % to 0.05 % of aldehyde impurities, formaldehyde and acetaldehyde.

## Patentansprüche

1. Verfahren zur Abtrennung von Ethylenoxid von Formaldehyd und Acetaldehyd aus unreinem Ethylenoxid, das Wasser und die genannten Aldehyde als Verunreinungen enthält, durch Destillation des unreinen Ethylenoxids in einer Rückflußkolonne, dadurch gekennzeichnet, daß die Destillation unter solchen Bedingungen durchgeführt wird, daß der am Sumpf der Kolonne austretende Flüssigkeitsstrom das im unreinen Ethylenoxid vorliegende Wasser und Ethylenoxid in der 0,15 bis 3-fachen Gewichtsmenge des Wassers enthält, während das aus der Trennung von Formaldehyd und Acetaldehyd stammende Ethylenoxid am Kopf der Kolonne entweicht.

2. Verfahren nach Anspruch 1, dadurch gekennzeichnet, daß das Gewichtsverhältnis der Ethylenoxidmenge zu der des Wassers in dem am Sumpf der Kolonne austretenden Flüssigkeitsstrom zwischen 0,3 und 1,5 liegt.

3. Verfahren nach einem der Ansprüche 1 oder 2, dadurch gekennzeichnet, daß die Menge der aldehydischen Verunreinigungen, Formaldehyd und Acetaldehyd, in dem unreinen Ethylenoxid, bezogen auf das Ethylenoxid 0,005 bis 0,02 Gew.% beträgt.

4. Verfahren nach irgendeinem der Ansprüche 1 bis 3, dadurch gekennzeichnet, daß das unreine Ethylenoxid eine Ethylenoxidmenge gleich oder größer 80 Gew.% enthält.

5. Verfahren nach irgendeinem der Ansprüche 1 bis 4, dadurch gekennzeichnet, daß das unreine Ethylenoxid eine Wassermenge von 0,5 Gew.% oder mehr enthält.

6. Verfahren nach irgendeinem der Ansprüche 1 bis 5, dadurch gekennzeichnet, daS die Kolonne bei einem mittleren absoluten Druck zwischen 2,5 und 5 bar betrieben wird.

7. Verfahren nach irgendeinem der Ansprüche 1 bis 6, dadurch gekennzeichnet, daß die Temperatur am Sumpf der Kolonne 40 bis 60°C beträgt.

8. Verfahren nach irgendeinem der Ansprüche 1 bis 7, dadurch gekennzeichnet, daß die Kolonne zwischen 30 und 60 theoretische Böden hat.

9. Verfahren nach irgendeinem der Ansprüche 1 bis 8, dadurch gekennzeichnet, daS das unreine Ethylenoxid in die Kolonne in einer Höhe zwischen der Hälfte und drei Vierteln der theoretischen Böden, gerechnet vom Kopf der Kolonne eintritt.

10. Verfahren nach Anspruch 9, dadurch gekennzeichnet, daß der Rückfluß aus einer kondensierten Fraktion des von den aldehydischen Verunreinigungen befreiten Ethylenoxids besteht, wobei das Gewichtsverhältnis des zurückfließenden Ethylenoxids zu dem nicht-zurückgeführten zwischen 1 und 9 beträgt.

11. Verfahren nach Anspruch 10, dadurch gekennzeichnet, daß das Gewichtsverhältnis von zurückfließendem Ethylen zu dem nicht-zurückgeführten zwischen 1,5 und 6 beträgt.

12. Verfahren zur Isolierung eines von aldehydischen Verunreinigungen befreiten Ethylenoxids aus dem Gasstrom der katalytischen Gasphasenoxidation von Ethylen mit Sauerstoff, umfassend
(a) - eine Absorption in Wasser durch In-Berührung-Bringen des genannten Gasstroms mit einem im wesentlichen wäßrigen Strom, um eine verdünnte Lösung von Ethylenoxid zu erhalten,
(b) - eine Desorption, bei der die nach (a) erhaltene verdünnte wäßrige Ethylenoxidlösung einem Strippen mit Wasserdampf in einer Kolonne unterworfen wird, die bei einem mittleren absoluten Druck zwischen 1,5 und 6 bar arbeitet, um am Fuß der Kolonne einen wäßrigen, im wesentlichen von Ethylenoxid befreiten Strom und am Kopf der Kolonne einen Gasstrom zu erhalten, der Ethylenoxid enthält und nacheinander in zwei oder drei in Serie geschalteten Wärmeaustauschern bei einem absoluten mittleren Druck zwischen 1,5 und 6 bar kondensiert wird, wobei im letzten der Wärmeaustauscher die Temperatur des Kühlmediums zwischen 5°C und einer Höchsttemperatur liegt, die 5°C unter der Kondensationstemperatur von reinem Ethylenoxid bei dem angewendeten Druck liegt,
(c) - eine Destillation des den letzten der Ionenaustauscher gemäß (b) verlassenden Flüssigkeitsstromes
dadurch gekennzeichnet, daß (c) nach irgendeinem der Ansprüche 1 bis 11 durchgeführt wird, wobei das unreine Ethylenoxid der Flüssigkeitsstrom, aus dem letzten der zwei oder drei Wärmeaustauscher gemäß (b) ist.

13. Verfahren nach Anspruch 12, dadurch gekennzeichnet, daß das unreine Ethylenoxid 95 Gew.% Ethylenoxid oder mehr und 0,005 bis 0,05 % der aldehydischen Verunreinigungen, Formaldehyd und Acetaldehyd, enthält.
